**Europäisches Patentamt**

**European Patent Office**

**Office européen des brevets**

(11) Veröffentlichungsnummer : **0 645 135 A1**

(12) **EUROPÄISCHE PATENTANMELDUNG**

(21) Anmeldenummer : **94810557.2**

(22) Anmeldetag : **26.09.94**

(51) Int. Cl.⁶ : **A61K 7/42**

(30) Priorität : **29.09.93 CH 2929/93**

(43) Veröffentlichungstag der Anmeldung :
**29.03.95 Patentblatt 95/13**

(84) Benannte Vertragsstaaten :
**AT BE CH DE DK ES FR GB GR IE IT LI LU MC
NL PT SE**

(71) Anmelder : **Solco Basel AG**
**Gellertstrasse 18**
**CH-4052 Basel (CH)**

(71) Anmelder : **GREITER AG**
**Trogenerstrasse 80**
**CH-9450 Altstätten (CH)**

(72) Erfinder : **Billia, Mario**
**Grossackerstrasse 32**
**CH-9542 Münchwilen (CH)**
Erfinder : **Siladji, Sigrun**
**Frauenackerstrasse 7**
**CH-8356 Ettenhausen (CH)**

(74) Vertreter : **Zimmermann, Hans, Dr. et al**
**A. Braun Braun Héritier Eschmann AG**
**Holbeinstrasse 36-38**
**CH-4051 Basel (CH)**

(54) **Hämodialysat enthaltendes Sonnenschutzmittel.**

(57)  Mit einem Sonnenschutzmittel, welches neben kosmetischen Hilfsstoffen und Sonnenschutzfilter und/oder Sonnenblocker eine wirksame Menge eines deproteinierten Hämodialysates aus dem Blut von Säugetieren oder einer aktiven Fraktion davon enthält, wird eine Verbesserung des Zustandes der Haut und eine Verringerung oder Vermeidung der negativen Folgen der Sonnenexposition erreicht. Das Mittel eignet sich sowohl zur Prophylaxe als auch zur Nachbehandlung.

EP 0 645 135 A1

Die Erfindung betrifft ein Sonnenschutzmittel, welches mindestens eine als Sonnenschutzfilter und/oder Sonnenblocker wirkende Substanz und ein deproteiniertes Hämodialysat aus dem Blut von Säugetieren oder eine aktive Fraktion davon enthält, sowie dessen Herstellung.

Vermehrter Tourismus mit verstärkter Sonnenbelastung und verschlechterte Umweltbedingungen (Ozonloch) erhöhen das Risiko aktinischer Hautschädigungen, zu dessen Verminderung in Lichtschutzmitteln und Kosmetika physikalisch oder chemisch wirkende Substanzen eingesetzt werden. Ein vollumfänglicher Schutz wird aber nicht erreicht.

Die vermehrte und verlängerte Sonnenexposition, z.B. als Folge veränderter Freizeitaktivitäten, zeitigt Gefahren sowohl akuter als auch chronischer Art. Die Dosis der UV-Strahlung und deren Wellenlänge bestimmt die positiven oder negativen Folgen. Sonnenbrand und auch photoallergische Reaktionen sind häufig auftretende kurzfristige Folgen zu langer ungeschützter Sonneneinstrahlung. Zu den negativen Spätfolgen zählen frühzeitige Hautalterung und schliesslich auch Hautkrebs. Die Haut baut sich mit der Bildung von Melanin und der Verdickung der Hornschicht einen natürlichen eigenen Schutz gegen Sonnenstrahlen auf. Diese Reaktionen verlaufen in einer gesunden Haut und in gesunden Zellen optimal. In der als Folge mehrfacher Sonnenexposition geschädigten und frühzeitig gealterten Haut sind sie reduziert. Auch die Widerstandskraft gegen äussere Umwelteinflüsse, wie Wasser und Wind, ist geschwächt. Symptome wie Hautfalten, trockene, rauhe und ledrige Farmerhaut und Altersflecken treten bei der strapazierten Haut auf.

Es ist bekannt, dass ultraviolette Strahlung im Wellenlängenbereich von etwa 290 - 320 $\mu$m (UV-B-Bereich) rasch zu Symptomen wie Rötung oder Erythemen, Oedemen und Blasenbildung und nach verlängerter Exposition zu aktinischen Keratosen und Karzinomen führen kann. In den letzten Jahren wurden vermehrt Bedenken geäussert, dass auch ultraviolette Strahlung im Wellenbereich von etwa 320 - 400 $\mu$m (UV-A-Bereich) die Haut schädigen kann und zur frühzeitigen Alterung der Haut beiträgt.

Sonnenschutzfilter oder Sonnenblocker, die gegen die genannten Folgeschäden in Sonnenschutzmitteln und Kosmetika eingesetzt werden, absorbieren oder reflektieren die Sonnenstrahlen in diesen Wellenlängenbereichen. Eine direkte anabolische Wirkung auf die zellulären Mechanismen in der Haut wird damit aber nicht erreicht.

Anderseits ist bekannt, z.B. aus DE-PS 1 076 888, GB-PS 824 375 und EP-A-0 095 682, dass deproteinierte Hämodialysate aus dem Blut von Säugetieren oder aktive Fraktionen davon die Zellatmung und den Zellstoffwechsel stimulieren und zur Wundheilung geeignet sind. Solche Präparate werden von der Firma Solco Basel AG unter dem Namen Solcoseryl vertrieben.

Ueberraschenderweise wurde nun gefunden, dass die negativen Folgen der Sonnenexposition vermieden oder zumindest verringert werden können, wenn einem Sonnenschutzmittel ein solches Hämodialysat zugesetzt wird.

Die Erfindung betrifft daher ein Sonnenschutzmittel enthaltend eine wirksame Menge eines deproteinierten Hämodialysates aus dem Blut von Säugetieren oder einer aktiven Fraktion davon, ein oder mehrere als Sonnenschutzfilter und/oder Sonnenblocker wirkende Substanzen in der zur Erzielung eines Sonnenschutzfaktors von 2 bis 25 benötigten Menge und kosmetische Hilfsstoffe.

Der Ausdruck Sonnenschutzmittel bezeichnet im Rahmen der vorliegenden Erfindung Zusammensetzungen, die als externe Lichtschutzmittel zum Schutz der Haut vor ultravioletter Strahlung aufgebracht werden. Der Ausdruck Sonnenschutzfilter oder Lichtschutzfilter bezeichnet Substanzen, insbesondere organische Substanzen, die ultraviolettes Licht absorbieren und als energieärmere, längerwellige Strahlung wieder abgeben. Der Ausdruck Sonnenblocker bezeichnet Substanzen, insbesondere anorganische Substanzen und Pigmente, die nach Applikation auf der Haut eine physikalische undurchlässige Barriere für alle Photonen der Sonnenstrahlung bilden.

Der Gehalt an Hämodialysat oder einer aktiven Fraktion davon in den erfindungsgemässen Zusammensetzungen bewirkt eine eindeutige Verbesserung des Zustandes der Haut sowohl bei präventiver Anwendung als auch im Aftersun-Bereich. Die erfindungsgemässen Sonnenschutzmittel eignen sich daher zur Konditionierung, Prophylaxe und Nachbehandlung. Im Gegensatz zu den üblichen Sonnenschutzmitteln wird die Zellmigration und Zellproliferation gefördert und der hauteigene Abwehrmechanismus gegen die schädigende Wirkung der Sonnenstrahlen verbessert. Bei einer Dermatitis solaris wird die Zell- und Hautregeneration beschleunigt und eine Schuppung verhindert. Das Risiko der Bildung von Erythemen, Oedemen und Blasen, die Alterung der Haut und die Faltenbildung als Folge verlängerter Sonnenexposition werden deutlich verringert. Die erfindungsgemässen Präparate helfen somit die unerwünschten Merkmale gealterter und sonnenexponierter Haut weitgehend zu vermeiden.

Im Vergleich zu Sonnenschutzmitteln ohne Hämodialysat wird der Sonnenschutzfaktor der erfindungsgemässen Zusammensetzungen durch den Gehalt an deproteiniertem Hämodialysat oder einer Fraktion davon überraschenderweise erhöht, wobei mit zunehmender Sonnenexposition ein zunehmender Einfluss dieser Komponente festgestellt wurde.

Die Erfindung betrifft ebenfalls ein Verfahren zur Herstellung des neuen Sonnenschutzmittels, welches dadurch gekennzeichnet ist, dass man eine wirksame Menge eines deproteinierten Hämodialysates aus dem Blut von Säugetieren oder einer aktiven Fraktion davon, ein oder mehrere als Sonnenschutzfilter und/oder Sonnenblocker wirkende Substanzen in der zur Erzielung eines Sonnenschutzfaktors von 2 bis 25 benötigten Menge und die kosmetischen Hilfsstoffe miteiander vermischt. Die Herstellung kann in an sich bekannter Weise erfolgen beispielsweise dadurch, dass man zuerst die kosmetischen Hilfsstoffe vermischt und anschliessend Sonnenschutzfilter und/oder Sonnenblocker und Hämodialysat bzw. eine Fraktion davon in die kosmetische Grundlage einarbeitet.

Geeignete deproteinierte Hämodialysate und Fraktionen davon und geeignete Methoden zu deren Herstellung sind dem Fachmann bekannt. Die Herstellung kann aus Blut, Blutplasma, Serum, Blutzellen oder Organhomogenisaten von Säugetieren erfolgen. Als besonders geeignetes Ausgangsmaterial hat sich das Blut von jungen Schlachttieren, insbesondere von Kälbern, erwiesen.

Die Herstellung kann beispielsweise nach den in DE-PS 1 076 888 und GB-PS 824 375 bekannten Methoden durch enzymatischen Eiweissabbau und/oder durch Deproteinierung mittels Behandlung mit einem niedermolekularen aliphatischen Alkohol oder mit Säuren und anschliessendem Aufkonzentrieren erfolgen. Gemäss einer bevorzugten, aus DE-PS 3 042 999 bekannten Methode kann das Hämodialysat auch dadurch erhalten werden, dass man das Blut unter Zuhilfenahme einer semipermeablen Membran mit einer Durchlassgrösse für Molekulargewichte bis maximal 5000 gegen Wasser dialysiert, das Dialysat bei maximal 38°C auf 30 - 60 mg Trockensubstanz pro ml einengt und mit etwa 1 g Kieselgel auf 1 ml eingeengtes Dialysat mischt, das Gemisch filtriert, das beladene Kieselgel mit Methanol eluiert und das Eluat trocknet. Das auf diese Weise erhaltene trockene Dialysat vermeidet die Anwesenheit organischer Salze, die bei kosmetischen Anwendungen oft unerwünscht sind. Die Herstellung aktiver Fraktionen ist dem Fachmann ebenfalls bekannt. Beispielsweise wird in EP-A-0 095 682 die Isolierung von Glykolipiden mittels Autolyse und Dialyse gegen ein alkoholischwässriges Medium, anschliessender Ethanolausfällung oder Extraktion und Chromatographie beschrieben.

Das deproteinierte Hämodialysat kann als wässriges Dialysat, z.B. mit einem Gehalt an Trockensubstanz von 10 - 60 mg/ml, oder als Trockensubstanz verwendet werden. Die erfindungsgemässen Sonnenschutzmittel können das Hämodialysat oder eine aktive Fraktion davon vorzugsweise in vollentsalzter oder teilentsalzter Form enthalten.

Der Gehalt an deproteiniertem Hämodialysat oder einer aktiven Fraktion davon in den erfindungsgemässen Sonnenschutzmitteln kann je nach Formulierung etwa 0,1 bis etwa 2,0 Gew.-%, berechnet als Trockensubstanz, bezogen auf die gesamte Zusammensetzung, oder gewünschtenfalls auch mehr betragen. Im allgemeinen ist ein Gehalt von etwa 0,4 bis 0,8 Gew.-% bevorzugt.

Als Sonnenblocker eignen sich übliche anorganische Substanzen und Pigmente wie Kaolin, Zinkoxid, Talkum, Bentonit, Calciumcarbonat, Magnesiumoxid, Titandioxid, Eisenoxid, Magnesiumsilikat, Perlglanz und dergleichen. Sie reflektieren und streuen Sonnenlicht im ultravioletten, sichtbaren und infraroten Bereich und werden nicht resorbiert.

Da es äusserst erwünscht ist, dass das Sonnenschutzmittel nach dessen Anwendung auf der Haut unsichtbar ist, sollte die Teilchengrösse der Sonnenblocker vorzugsweise so gewählt werden, dass die Streuung des sichtbaren Lichts minimiert und die Undurchlässigkeit von UV-A und UV-B maximiert wird. Die erfindungsgemässen Sonnenschutzmittel enthalten daher vorzugsweise Mikropigmente, d.h. Pigmente mit sehr kleiner Teilchengrösse von beispielsweise weniger als 100 µm.

Gemäss einer besonders bevorzugten Ausführungsform enthalten die erfindungsgemässen Sonnenschutzmittel Zinkoxid und/oder Titandioxid als Sonnenblocker. Diese wirken nicht irritierend und ergeben insbesondere keine allergischen Reaktionen in den Augen und auf den Lippen, womit ein optimaler Sonnenschutz des ganzen Körpers ermöglicht wird. Vorzugsweise beträgt die Teilchengrösse von Titandioxid weniger als 35 µm, besonders bevorzugt weniger als 10 µm, und die Teilchengrösse von Zinkoxid weniger als 50 µm, besonders bevorzugt weniger als 20 µm. Solche Mikropigmente und deren Verwendung sind z.B. aus EP-A-0 433 086 bekannt.

Als Sonnenschutzfilter eignen sich übliche Substanzen, insbesondere Dibenzoylmethane, Benzophenone, p-Aminobenzoesäuren und/oder deren Ester, N-substituierte p-Aminobenzoesäure-Derivate, Campherderivate, Zimtsäureester und Benzimidazol-Derivate. Beispiele geeigneter Verbindungen sind p-Aminobenzoesäureglycerolester, p-Dimethylaminobenzoesäure-isoamylester, 2-Hydroxy-4-octyloxybenzophenon, 2-Hydroxy-4-methoxybenzophenon-5-sulfonsäure-trihydrat, 4-Phenylbenzophenon-2'-carbonsäure-2-ethylhexylester, 4-Phenylbenzophenon, 2-Hydroxy-4-methoxybenzophenon, 2,2'-Dihydroxy-4,4'-dimethoxybenzophenon, 2,2',4,4'-Tetrahydroxybenzophenon, 2,2'-Dihydroxy-4,4'-dimethoxybenzophenon-5-natriumsulfonat, p-Methoxyzimtsäurepropylester, p-Methoxyzimtsäure-2-ethoxyethylester, p-Methoxyzimtsäure-2-ethylhexylester, p-Methoxyzimtsäure-isoamylester, p-Methoxyzimtsäure-diethanolaminsalz, 3,4-dimethoxyphenylglyoxylsaures Natrium, 2-Phenylbenzimidazol-5-sulfonsäure, 5-Methyl-2-phenylbenzoxazol, 3-(4-Methylbenzyliden)-campher, Dibenzalazin, 5-(3,3-Dimethyl-2-norbornyliden)-3-penten-2-on, Dianisoylmethan und dergleichen. Diese Verbindungen absorbieren aufgrund ihrer Struktur, z.B. einer ausreichenden Anzahl konjugierter Doppelbindungen, ultraviolettes Licht in einem für sie charakteristischen Wellenlängenbereich und werden je nach Wellenlängenbereich als Breitbandfilter, UV-B-Filter oder UV-A-Filter bezeichnet. Die erfindungsgemässen Sonnenschutzmittel können Breitbandfilter, UV-B-Filter und/oder UV-A-Filter enthalten.

Die Sonnenschutzmittel gemäss der vorliegenden Erfindung können nur Sonnenblocker, nur Sonnenschutzfilter oder eine Kombination beider enthalten. Der Gehalt an Sonnenblocker und/oder Sonnenschutzfilter hängt vom gewünschten Sonnenschutzfaktor ab und kann vom Fachmann von Fall zu Fall leicht ermittelt werden.

Die erfindungsgemässen Sonnenschutzmittel können in üblichen Anwendungsformen, insbesondere in Form von W/O-und O/W-Crèmen (Wasser in Oel bzw. Oel in Wasser), Gelen, Lipo-Gelen, Lotionen, Spray-Lösungen, Salben, Pasten und dergleichen, zur Anwendung gelangen. Diese Anwendungsformen können in an sich bekannter Weise und unter Verwendung üblicher Arten und Mengen von kosmetischen Hilfsstoffen hergestellt werden. Geeignete Hilfsstoffe sind dem Fachmann bestens bekannt.

Die erfindungsgemässen Sonnenschutzmittel können neben den zur Bildung einer kosmetischen Grundlage je nach Anwendungsform jeweils benötigten Komponenten insbesondere auch Emulgatoren, Antioxidantien, Radikalfänger, Konservierungsmittel, schutzfilmbildende Komponenten, entzündungshemmende Komponenten, Komponenten mit bakteriostatischer oder fungizider Wirksamkeit, geruchsgebende Komponenten (insbesondere Duftstoffe), die Wasserfestigkeit verbessernde Komponenten, Salze (z.B. Natriumchlorid), Vitamine und/oder Pigmente enthalten. Derartige Zusätze sind dem Fachmann ebenfalls bestens bekannt.

Die Erfindung wird durch die folgenden Formulie rungsbeispiele weiter veranschaulicht. Die Herstellung erfolgte jeweils durch Vermischen der Hilfsstoffe in bekannter Weise und anschliessendes Einarbeiten der Sonnenblocker und/oder Sonnenschutzfilter und des Hämodialysats in die kosmetische Grundlage. Als Hämodialysat wurde jeweils ein deproteiniertes Hämodialysat aus Kälberblut mit einem Gehalt an Trockensubstanz von 40 mg/ml verwendet.

Beispiel 1

Sonnenschutzcrème (Wasser in Oel) mit organischen UV-Filtern und einem Sonnenschutzfaktor von 20 aus:

|  | Gew.-% |
|---|---|
| Wasser | 47 |
| Hämodialysat (Trockengehalt: 40 mg/ml) | 19 |
| 1,3-Bis-(2-ethylhexyl)-cyclohexan | 8 |
| p-Methoxyzimtsäure-octylester | 7,5 |
| Mineralöl | 5 |
| Octylstearat | 3 |
| Propylenglykol | 2,25 |
| Cetyldimethicon Copolyol | 2 |
| 1-(4-Methoxyphenyl)-3-(4-tert.-butylphenyl)-propan-1,3-dion | 2 |
| 3-(4-Methylbenzyliden)-campher | 1,5 |
| Polyglyceryl-4-isostearat | 1 |
| 2-Hydroxy-4-methoxybenzophenon | 0,5 |
| Duftstoffe | 0,5 |
| Natriumchlorid | 0,3 |
| EDTA | 0,2 |
| Methylparaben | 0,175 |
| Propylparaben | 0,075 |

Beispiel 2

Sonnenschutzlotion mit organischen UV-Filtern und einem Sonnenschutzfaktor von 6 aus:

|  | Gew.-% |
|---|---|
| Wasser | 57,2 |
| Hämodialysat (Trockengehalt: 40 mg/ml) | 10 |

| | |
|---|---|
| Mineralöl | 8 |
| Glyceride der $C_8$-$C_{10}$-Fettsäuren | 6 |
| Cetyldimethicon-Copolyol/Hexyllaurat | 5 |
| p-Methoxyzimtsäure-octylester | 4 |
| Isopropylpalmitat | 3 |
| Propylenglykol | 2,25 |
| 1-(4-Methoxyphenyl)-3-(4-tert.-butylphenyl)-propan-1,3-dion | 1 |
| Tocopherylacetat | 1 |
| 2-Hydroxy-4-methoxybenzophenon | 0,5 |
| Duftstoffe | 0,5 |
| Siliciumdioxid | 0,5 |
| Natriumchlorid | 0,5 |
| Tocopherol und hydriertes Talgglyceridcitrat | 0,3 |
| Methylparaben | 0,175 |
| Propylparaben | 0,075 |

## Beispiel 3

Sonnenschutzlotion mit Zinkoxid und einem Sonnenschutzfaktor von 15 aus:

| | Gew.-% |
|---|---|
| Wasser | 34,2 |
| Zinkoxid | 20 |
| Hämodialysat (Trockengehalt: 40 mg/ml) | 19 |
| Cetyldimethicon-Copolyol/Hexyllaurat | 5 |
| Glyceride der $C_8$-$C_{10}$-Fettsäuren | 5 |
| Mineralöl | 4 |
| Isopropylstearat | 3 |
| Stearyldimethicon | 3 |
| Propylenglykol | 2,5 |
| Petrolat | 2 |
| Sorbitol | 1,5 |
| Duftstoffe | 0,5 |
| Natriumchlorid | 0,3 |

## Beispiel 4

Sonnenschutzcrème (Wasser in Oel) mit Zinkoxid und Titandioxid, mit einem Sonnenschutzfaktor von 10, aus:

|  | Gew.-% |
|---|---|
| Wasser | 55,9 |
| Hämodialysat (Trockengehalt: 40 mg/ml) | 10 |
| Zinkoxid | 10 |
| Mineralöl | 5 |
| Isopropylpalmitat | 3,5 |
| Methylglucosid-dioleat | 3 |
| Methylgluceth-20 | 3 |
| Titandioxid | 3 |
| Propylenglykol | 2,5 |
| PEG-45/Dodecylglykol-Copolymer | 1 |
| Mineralöl und Aluminium-Magnesium-Hydroxystearat | 1 |

Jojobaöl {
C38 flüssige Ester:- 6%
C40 flüssige Ester:- 30%
C42 flüssige Ester:- 49%     1
C44 flüssige Ester:- 8%
Fraktionstotal flüssige Ester:- 96%

| Magnesiumsulfat | 0,6 |
| Duftstoffe | 0,5 |
| Methylparaben | 0,175 |
| Propylparaben | 0,075 |

**Patentansprüche**

1. Sonnenschutzmittel enthaltend eine wirksame Menge eines deproteinierten Hämodialysates aus dem Blut von Säugetieren oder einer aktiven Fraktion davon, ein oder mehrere als Sonnenschutzfilter und/oder Sonnenblocker wirkende Substanzen in der zur Erzielung eines Sonnenschutzfaktors von 2 bis 25 benötigten Menge und kosmetische Hilfsstoffe.

2. Sonnenschutzmittel nach Anspruch 1, dadurch gekennzeichnet, dass es das deproteinierte Hämodialysat oder eine aktive Fraktion davon in vollentsalzter oder teilentsalzter Form enthält.

3. Sonnenschutzmittel nach Anspruch 1 oder 2, dadurch gekennzeichnet, dass der Gehalt an deproteiniertem Hämodialysat oder einer aktiven Fraktion davon 0,1 bis 2,0 Gew.-%, berechnet als Trockensubstanz, bezogen auf die gesamte Zusammensetzung, beträgt.

4. Sonnenschutzmittel nach Anspruch 3, dadurch gekennzeichnet, dass der Gehalt an deproteiniertem Hämodialysat oder einer aktiven Fraktion davon 0,4 bis 0,8 Gew.-%, berechnet als Trockensubstanz, bezogen auf die gesamte Zusammensetzung, beträgt.

5. Sonnenschutzmittel nach einem der Ansprüche 1 bis 4, dadurch gekennzeichnet, dass es Zinkoxid, Titandioxid, Kaolin, Talkum, Bentonit, Calciumcarbonat, Magnesiumoxid, Eisenoxid, Magnesiumsilikat und-/oder Perlglanz als Sonnenblocker enthält.

6. Sonnenschutzmittel nach einem der Ansprüche 1 bis 5, dadurch gekennzeichnet, dass es als Sonnenblocker ein oder mehrere Mikropigmente, vorzugsweise Zinkoxid mit einer Teilchengrösse von weniger als 50 μm und/oder Titandioxid mit einer Teilchengrösse von weniger als 35 μm, enthält.

7. Sonnenschutzmittel nach einem der Ansprüche 1 bis 6, dadurch gekennzeichnet, dass es mindetens ein Breitbandfilter, UV-B-Filter und/oder UV-A-Filter enthält.

8. Sonnenschutzmittel nach Anspruch 7, dadurch gekennzeichnet, dass es als Sonnenschutzfilter mindestens ein Dibenzoylmethan, ein Benzophenon, eine p-Aminobenzoesäure und/oder einen Ester davon, ein N-substituiertes p-Aminobenzoesäure-Derivat, ein Campher-Derivat, einen Zimtsäureester und/oder ein Benzimidazol-Derivat enthält.

9. Verfahren zur Herstellung eines Sonnenschutzmittels gemäss Ansprüchen 1 bis 8, dadurch gekennzeichnet, dass man eine wirksame Menge eines deproteinierten Hämodialysates aus dem Blut von Säugetieren oder einer aktiven Fraktion davon, ein oder mehrere als Sonnenschutzfilter und/oder Sonnenblocker wirkende Substanzen in der zur Erzielung eines Sonnenschutzfaktors von 2 bis 25 benötigten Menge und die kosmetischen Hilfsstoffe miteinander mischt.

**Europäisches Patentamt**

# EUROPÄISCHER RECHERCHENBERICHT

Nummer der Anmeldung

EP 94 81 0557

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE-A-27 57 937 (BYK GULDEN LOMBERG) 28. Juni 1979<br><br>----- | 1-3 | A61K7/42 |
| | | | **RECHERCHIERTE SACHGEBIETE (Int.Cl.6)**<br><br>A61K |

Der vorliegende Recherchenbericht wurde für alle Patentansprüche erstellt

| Recherchenort | Abschlußdatum der Recherche | Prüfer |
|---|---|---|
| DEN HAAG | 9. Januar 1995 | Klaver, T |

EPO FORM 1503 03.82 (P04C03)